# EUROPEAN PATENT SPECIFICATION

(11) **EP 4 482 573 B1**
(45) Date of publication and mention of the grant of the patent: **01.04.2026**
(21) Application number: 23707507.2
(22) Date of filing: 21.02.2023
(51) Int. Cl.: A61N 5/10

(54) **POSITIONING SYSTEM FOR RADIOTHERAPY**
POSITIONIERUNGSSYSTEM FÜR STRAHLENTHERAPIE
SYSTÈME DE POSITIONNEMENT POUR LA RADIOTHÉRAPIE

(30) Priority: 22.02.2022 EP 22158005
(43) Date of publication of application: 01.01.2025
(73) Proprietor: RIJKSUNIVERSITEIT GRONINGEN, 9712 CP Groningen (NL); Academisch Ziekenhuis Groningen, 9713 GZ Groningen (NL)
(72) Inventor: BOTH, Stefan, 9713 GZ Groningen (NL); MARMITT, Gabriel Guterres, 9713 GZ Groningen (NL)
(74) Representative: V.O.
(86) International application number: PCT/NL2023/050085
(87) International publication number: WO 2023/163589

(56) References cited:
- WO-A1-2019/169455
- WO-A1-2021/184107
- XIN ZHEN ET AL: "Deep convolutional neural network with transfer learning for rectum toxicity prediction in cervical cancer radiotherapy: a feasibility study", PHYSICS IN MEDICINE AND BIOLOGY, INSTITUTE OF PHYSICS PUBLISHING, BRISTOL GB, vol. 62, no. 21, 11 October 2017 (2017-10-11), pages 8246 - 8263, XP020321197, ISSN: 0031-9155, [retrieved on 20171011], DOI: 10.1088/1361-6560/AA8D09

## Description

### Field

The present invention pertains to a positioning system for radiotherapy.

The present invention further pertains to a training system.

The present invention still further pertains to a training method.

### Background

Radiotherapy (also called radiation therapy) is a cancer treatment that uses high doses of ionizing radiation to kill cancer cells and shrink tumors. As a preparation for the treatment, a radiation oncologist in collaboration with a medical physicist and a radiation therapist designs a treatment plan that aims to result in an effective irradiation of malignant area(s), while minimizing exposure to healthy tissue.

The radiation oncologist bases the treatment plan on the location of the malignant area(s) from a 3D/4D CT image of the patient obtained prior to the treatment. In this connection it is noted that throughout the description the wording "image" is used to denote a volumetric image data set or scan obtained from a volumetric imaging method. Non-limiting examples of volumetric imaging methods are CT, CBCT, PET, MRI, and synCT. During the course of treatment, which usually comprises multiple treatment sessions the actual situation may differ from the situation as observed in the 3D/4D-image. This may be due to various causes, such as the position of the patient, a change in shape of the tumor, a loss or gain of weight of the patient and internal movements resulting from breathing and the patient's heart beat.

Haehnle et al, 2017 Phys. Med. Biol. 62 165, describe a method for interactive multi-objective dose-guided patient positioning. The method proposed therein aims to reposition the patient such that an optimal dose distribution is achieved, i.e. a dose distribution that best matches the objectives of effective irradiation of malignant areas, while sparing healthy areas, in particular sparing organs at risk (OAR). The method proposed therein comprises a first step wherein a 3D cone-beam computed tomography (CBCT) scan is obtained with the patient in the treatment position. In the first step a rigid alignment is computed for which the anatomical structures of the patient of the original image best coincide the anatomical structures in the CBCT scan. In a second step, dose distributions are computed that would be achieved when applying the original treatment plan to the patient with this rigid realignment and for multiple alignments different from this alignment within a space C of accessible shifts. Furthermore dose distributions for intermediate alignments are computed by interpolation. It is a disadvantage of the known method that the dose computation for the various alignments imposes a substantial computational burden and involves a substantial computation time.

Another system and method for patient positioning is described in publication WO 2021/184107 A1.

### SUMMARY

It is an object of the present invention to provide an improved positioning system requiring less computational resources.

In accordance therewith an improved positioning system is provided as defined in claim 1.

The improved system claimed therein positions the patient for treatment in a beam of radiation in accordance with patient specific data including a first image of the patient. The first image is typically a 3D/4D- image, obtained with a volumetric imaging method as mentioned above, for example obtained with a CT or and less frequently an MRI or PET/CT scanner. The patient specific data is provided by a medical specialist, for example an oncologist, for example using a planning tool. During the treatment planning process the target and organs at risk are segmented, the adequate number of beam orientations or arc lengths are established relative to the isocenter, and based on the physician prescribed dose to tumor and objectives for organs at risk, the plan is optimized and calculated to determine the best dose distribution for a particular patient. The treatment plan is typically delivered over several weeks of treatment, 5 days a week.

The improved system further comprises an imaging device that is configured to provide a second image(scan) of the patient including the part of the body to be treated Also the imaging device for providing the second image is typically a 3D-imaging device like a CT-scanner or an MRI scanner. The second image provides more recent information about the location and sizes of tissues and structures in the body of the patient, preferably immediately prior to a treatment session.

The improved system further comprises a positioning device such as a treatment couch for holding the patient in a variable position and/or orientation in the beam of radiation for an accurate intervention to a designated part of the patient. The positioning device is controlled by an optimization controller comprised in the improved system. The optimization controller is configured to provide registration control data, i.e. control data that controls the position and/or orientation of the positioning device to guide the positioning device based on the first image and the second image. The optimization controller comprises a dose based control module that is configured to compute an optimized repositioning of the patient by performing a dose-guided optimization. To that end the dose based control module is configured to perform at least the following operations.

It performs neuromorphic data processing operations to first image data obtained from the first image to render first feature map data. It also performs the same neuromorphic data processing operations to second image data obtained from the second image to render second feature map data. Subsequently it concatenates the first feature map data and the second feature map data to provide concatenated feature map data. It then performs further neuromorphic data processing operations to the concatenated feature map data to provide a correction vector indicative for a required registration of the patient for dose based optimization. The concatenated feature map data is for example a feature map comprising both the first feature map data and the second feature map data. Due to the fact that the steps of the method are performed in this order, i.e. first respective feature map data is extracted from each image and the feature map data so obtained is then concatenated it is rendered possible that the same neural network architecture can be trained and used with different types of images, such as CBCT/MRI. For this reason, registration between different image types (CT+CBCT or CT+MRI) could be performed by the same neural network.

As specified in more detail below, a dose based control module as specified for the improved system can be easily trained to perform the registration in an efficient manner.

In an embodiment, the improved system further comprises a geometry based control module and a transformation module. The geometry based control module is configured to perform a geometrical rigid registration to provide a first estimate for a required registration of the patient based on the first image and the second image. This geometrical rigid registration is based on a registration of rigid structures in the body of the patient, such as bones, or markers that are deliberately introduced at the time of planning the treatment. This geometrical rigid registration would be sufficient in the hypothetical case that no morphological changes occur in the body of the patient. In practice however, such morphological changes do occur, for example due to change of weight and changes in the size of the tumor to be treated. Accordingly, the geometry based control module is provided in addition to the dose-based control module, but not as a replacement. The registration provided by the geometry based control module is however a first approximation that serves as a starting point for the dose-based control module. To compensate for a correction that is already provided by the first estimate, the transformation module is provided to transform either of the first image or the second image to compensate for a difference in registration as indicated by the first estimate. The dose based control module in this embodiment of the improved system is configured to determine an estimate for a required additional registration of the patient based on the first image and the second image of which one is transformed. For example, the transformation module transforms the second image in accordance with the first approximation as if the registration of the patient were already corrected in accordance with the first approximation, and the dose based control module determines the estimate for the required additional registration correction on the basis of the first image and the transformed second image. Alternatively, the transformation module transforms the first image in accordance with the inverse of the transformation specified by the first approximation, and the dose based control module determines the estimate for the required additional registration correction on the basis of the inversely transformed first image and the second image. It is an advantage of the embodiment of the improved system that further comprises a geometry based control module and a transformation module as described above that the dose based control module can be trained more easily. The geometry based control module and transformation module can be implemented in a straightforward manner requiring only modest computation effort.

In embodiments of the improved system the dose based control module comprises a first and a second, mutually identical convolutional neuromorphic processing branch, a neuromorphic concatenation stage and a fully connected neuromorphic stage.

In these embodiments, the first convolutional neuromorphic processing branch is configured to receive the first image data obtained from the first image and to provide the first feature map data based on the received first image data.

The second convolutional neuromorphic processing branch is configured to receive the second image data obtained from the second image and to provide the second feature map data based on the received second image data. It is noted that instead a single convolutional neuromorphic processing unit can be used in a time shared manner for computation of the first feature map data and the second feature map data from the first and the second image data respectively.

The neuromorphic concatenation stage is configured to receive the first feature map data and the second feature map data and to provide the concatenated feature map data based on the first feature map data and the second feature map data.

The fully connected neuromorphic stage is configured to receive the concatenated feature map data and to provide the correction vector indicative for the correction in the registration.

In exemplary embodiments the mutually identical convolutional neuromorphic processing branches each comprise a plurality of stages with a convolutional neuromorphic layer. Typically the mutually identical convolutional neuromorphic processing branches or the single convolutional neuromorphic processing branch applied in a time-shared manner comprises up to ten stages, e.g. 5 stages.

In addition to a convolutional neuromorphic layer the stages may comprise one or more of a batch normalization layer, an activation layer, e.g. a ReLU layer, for example a leaky ReLU activation layer, a pooling layer, e.g. a max pooling layer etc.

An embodiment of the improved system further comprises a third control module that is configured to compute a correction vector for dose-guided optimization by applying a gradient descent algorithm. Also in this embodiment the dose based control module is optionally combined with the geometry based control module.

In an example of this embodiment, the third control module comprises the following components.
a) A transformation vector modifier configured to generate a set of modified transformation vectors for each transformation vector at its input.
b) An image transformation module that is configured to provide a set of modified second images by transforming the second image in accordance with respective modified transformation vectors of the set of modified transformation vectors.
c) A dose simulation module configured to provide for each modified second image an estimated spatial dose distribution.
d) A dose volume histogram computation module to compute an expected dose volume histogram resulting for each estimated spatial dose distribution.
e) An evaluation module to compute a quality measure that indicates the expected quality of the treatment based on the computed expected dose volume histogram for each estimated spatial dose distribution.
f) A gradient descent module to compute a single next iterated transformation vector based on the respective values for the expected quality determined for the modified transformation vectors of the set of modified transformation vectors.

In operation the third control module iteratively computes a transformation vector until further iterations do not result in a substantial increase of the quality measure. Alternatively a predetermined maximum number of iterations may be specified. As a still further alternative the iteration stops as soon as one of these two conditions is met.

The simulation module in this example may estimate the spatial dose distribution with a Monte Carlo simulation.

The quality measure may additionally be based on an estimation of the normal tissue complication probability (NTCP), indicative for a probability that normal tissue is affected by the treatment. In an embodiment the third module further comprises a treatment effect computation module that provides an indication for this probability associated with the spatial dose distribution estimated by the dose simulation module. In this computation the treatment effect computation module may take into account one or more of RT-structure information and information about prognostic factors. In this embodiment the evaluation module is configured to compute the quality measure based on the computed expected dose volume histogram and based on the estimation of the normal tissue complication probability.

According to a second aspect of the present disclosure a training system for training a dose based control module is provided that comprises.
a) a first input for receiving a first image of a patient, the first image comprising tissue label data and dose specification data.
b) a second input for receiving a second image of the patient, the second image including a designated part of the patient to be treated and being taken at a point in time later than the first image.
c) a third input to receive a Golden Truth (GT) indication of a transformation vector specifying a correction of the position and/or orientation of the patient with which a dose delivered to the patient optimally approximates the dose as originally intended in accordance with the first image.
d) a transformation vector generator unit to generate a plurality of transformation vectors.
e) an image transformation unit to obtain respective transformed second images by applying the transformation vectors to the second image.
f) an addition unit which for each transformed second image computes the corresponding GT-correction vector from the GT-indication of the second image and the transformation vector with which the transformed second image was obtained.
g) a loss computation module to determine for each transformed second image a loss based on the difference between the GT-correction vector and a correction vector estimated by the dose based control module when the first image and the transformed second image are provided as input.
h) an update module for updating parameters of the dose based control module based on the computed loss.

The update module is for example configured to update the parameters of the dose based control module by a back propagation procedure.

In accordance with a third aspect of the present disclosure a method for training a neuromorphic dose based control module is provided that comprises the following operations.
a) Providing a first image of a patient comprising tissue label data and dose specification data.
b) Providing a second image of the patient including a designated part of the patient to be treated, therewith also providing a Golden Truth (GT) indication of a transformation vector specifying a correction (xc, yc, zc) of the position and/or orientation of the patient with which a dose delivered to the patient optimally approximates the dose as originally intended in accordance with the first image.
c) Generating an augmented set of second images by applying mutually different geometrical transformations, e.g. translations and/or rotations in three dimensional space to the second image and for each species of the augmented set of second images computing the Golden Truth for the required correction from the GT-indication of the second image and the transformation with which that species was obtained from the second image.
d) With the dose based control module computing a respective output vector indicative for a correction of the position and/or orientation of the patient for each pair of the first image and a species of the augmented set of second images.
e) For each species of the augmented set of second images computing a loss from the difference between the correction specified by the respective output vector and the respective GT.
f) Updating parameters of the dose based control module to reduce the loss. The training system according to the second aspect and training method according to the third aspect enable an efficient training of the dose based control module in that for each manually labeled second image a plurality of transformed second images are automatically generated that are used for training.

It is noted that an exemplary embodiment of the improved position correction system comprising the third control module is configured to update parameters of the dose based control module based on a loss defined by a difference between the correction vector determined by the third control module and the correction vector determined by the dose based control module. The loss defined by the difference is for example a distance measure, e.g. a block based distance measure (L1), an Euclidian distance measure (L2) or an L∝ distance measure. This renders it possible to perform a further training of the dose based control module even when it is already in use.

### BRIEF DESCRIPTION OF THE DRAWINGS

These and other aspects are shown in more detail with reference to the drawings. Therein:
FIG. 1 schematically shows an embodiment of a system for positioning a patient for treatment in a beam of radiation from a radiation source;
FIG. 2 shows a further embodiment;
FIG. 3 shows a component of that embodiment;
FIG. 4 shows an exemplary part of said component in further detail;
FIG. 5 schematically illustrates an embodiment of a method for training a neuromorphic dose based control module;
FIG. 6 schematically shows an embodiment of a training system for training the dose based control module;
FIG. 7 shows a further embodiment of the positioning system;
FIG. 8 shows an exemplary component of said embodiment in more detail;
FIG. 9 schematically shows a further embodiment of the system;
FIG. 10A, 10B, 11A-11D and 12A - 12D show experimental date obtained in the process of training the neural network of FIG. 4;
FIG. 13 shows further experimental data.

### DETAILED DESCRIPTION OF EMBODIMENTS

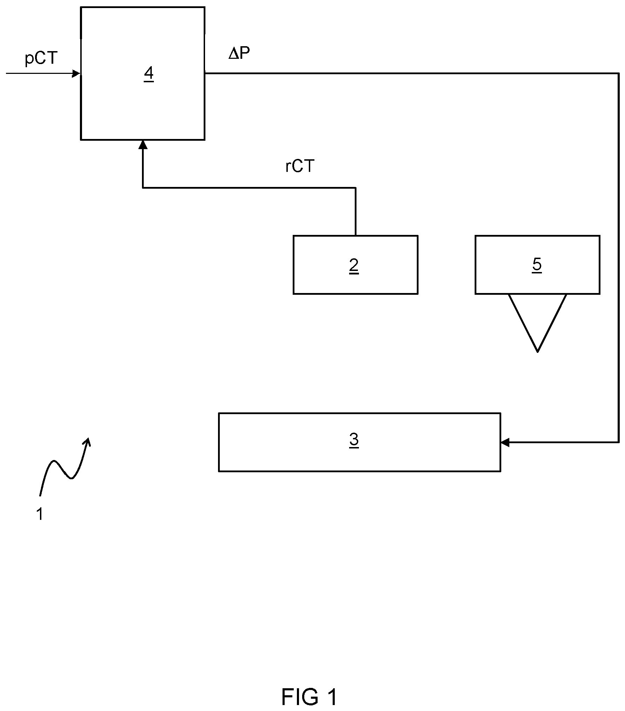

FIG. 1 schematically shows a system 1 for positioning a patient for treatment in a beam of radiation from a radiation source 5, for example a photon radiation source or a proton radiation source in accordance with patient specific data. The patient specific data includes a first image pCT of the patient that comprises tissue label data and dose specification data. As shown in FIG. 1, the system comprises an imaging device 2, a positioning device 3, and an optimization controller 4.

The positioning device 3, such as a robotic couch, is provided for holding the patient in a variable position in the beam of radiation from the radiation source 5 for an accurate intervention to a designated part of the patient.

The patient specific data is provided by an oncologist before the treatment of the patient and typically specifies a series of treatment sessions over a period of time. In accordance therewith the radiation source 5 is configured to issue a controlled beam of radiation with a predetermined distribution for a predetermined time.

Therewith it is necessary to properly register the patient. That is, the patient must be properly positioned in the beam of radiation. For that purpose the imaging device 2 is configured to provide a second image rCT of the patient that includes a designated part of the patient to be treated.

The optimization controller 4 is configured to provide positioning control data ΔP to guide the positioning device 3 based on the first image pCT and the second image rCT.

One approach is to register the patient such that positions of rigid structures in the body, such as bone tissue or markers identified in the second image match those of the positions in the first image pCT.

It is however not necessarily sufficient to simply position the patient in exactly the orientation as foreseen by the oncologist while preparing the treatment. This is due to the fact that the distribution of tissues of the body of the patient may have changed in the time interval between the date of the preparation of the treatment and the date of a treatment session. It may for example be the case that the patient has lost or gained weight or that the tumor which is treated has shrunken or instead increased in size.

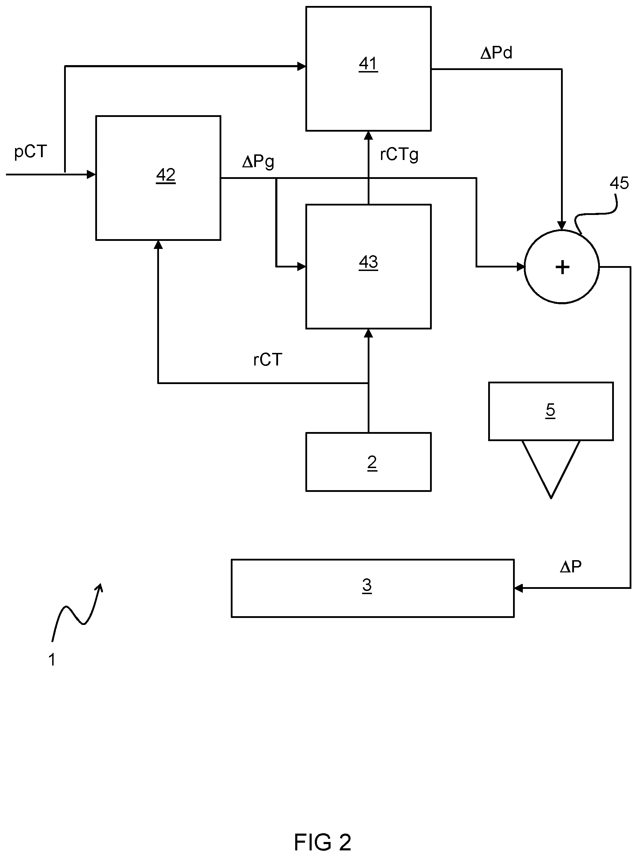

As shown in more detail in FIG. 2, to take into account such morphologic changes in time, the optimization controller 4 comprises a dose based control module 41 that is configured to compute a registration of the patient so that the applied dose optimally matches the dose as specified by oncologist in the treatment plan.

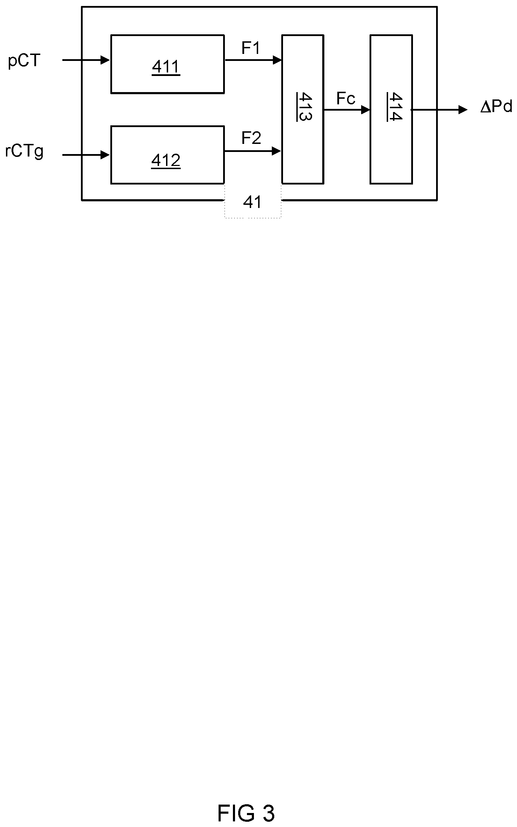

As shown in FIG. 3, in operation the dose based control module 41 performs neuromorphic data processing operations to first image data obtained from the first image pCT to render first feature map data F1. The dose based control module 41 in operation also performs the same neuromorphic data processing operations to second image data obtained from a second image rCTq to render second feature map data F2. The first image pCT and second image rCTq comprise voxelized volumetric x-ray data in DICOM files of CT modality.

Subsequent to these operations it concatenates the first feature map data F1 and the second feature map data F2 to provide concatenated feature map data Fc. Then it performs further neuromorphic data processing operations to the concatenated feature map data Fc to provide a correction vector ΔPd indicative for a required repositioning of the patient for dose based optimization.

In an embodiment the dose based control module 41 is configured to perform the specified operations with a suitably programmed general purpose processor. In the embodiment of FIG. 3, the dose based control module 41 comprises a first convolutional neuromorphic processing branch 411, a second convolutional neuromorphic processing branch 412 identical to the first convolutional neuromorphic processing branch 411, a neuromorphic concatenation stage 413 and a fully connected neuromorphic stage 414.

The first convolutional neuromorphic processing branch 411 is configured to receive the first image data obtained from the first image pCT and to provide the first feature map data F1 based on the received first image data. The second convolutional neuromorphic processing branch 412 is identical to the first convolutional neuromorphic processing branch 411 and is configured to receive the second image data obtained from the second image rCT and to provide the second feature map data F2 based on the received second image data. The neuromorphic concatenation stage 413 is configured to receive the first feature map data F1 and the second feature map data F2 and to provide concatenated feature map data Fc based on the first feature map data and the second feature map data. The fully connected neuromorphic stage 414 is configured to receive the concatenated feature map data Fc and to provide the output vector ΔPd indicative for the correction in the registration.

Referring again to FIG. 2, it is shown therein that the system 1 for positioning further comprises a geometry based control module 42 and a transformation module 43.

In operation, the geometry based control module 42 performs a geometrical rigid registration to provide a first estimate ΔPg for a required repositioning of the patient based on the first image pCT and the second image rCT. In the embodiment shown, the transformation module 43 in operation transforms the the second image to compensate for a difference in registration as indicated by the first estimate ΔPg. The dose based control module 41 in operation then determines an estimate ΔPd for a required additional repositioning of the patient based on the first image pCT and the transformed second image rCTg. In the example shown the dose based control module 41 adds (adder 45) the first estimate ΔPg from the geometry based control module 42 and the estimate ΔPd from the dose based control module 41 to compute the positioning control data ΔP for guiding the positioning device.

In an alternative embodiment the dose based control module 41 directly computes the positioning control data ΔP from the first image pCT and the second image rCT. This has the advantage that the transformation module 43 and the adder 45 are not required, which simplifies the system 1. The embodiment of the system as it is shown in FIG. 2 however has the advantage that training of the dose based control module 41 is simplified.

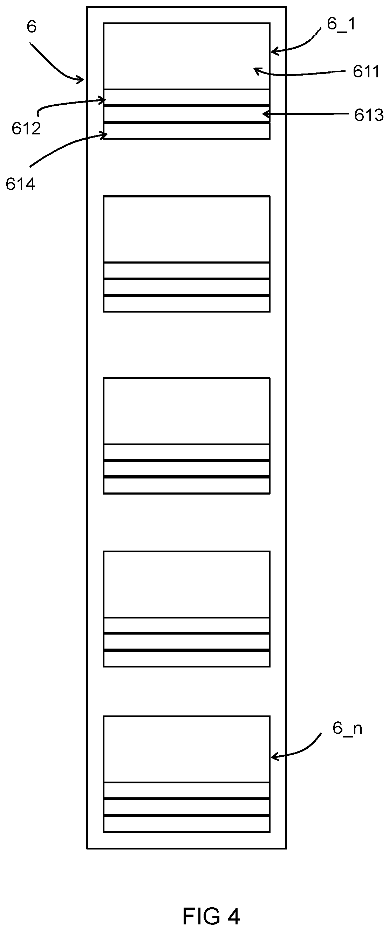

An exemplary structure of a convolutional neuromorphic processing unit 6 which may be used both for the convolutional neuromorphic processing branch 411 and the convolutional neuromorphic processing branch 412 is shown in FIG. 4. In the embodiment shown, the convolutional neuromorphic processing unit 6 comprises a plurality n of convolutional neuromorphic processing layers 6_1,...,6_n, for example a few to a few tens of layers. In the example shown, the selected value for n is equal to 5. Furthermore, in the example shown, a convolutional neuromorphic processing layer comprises a 3D-convolutional layer 611, a batch normalization layer 612, an activation layer 613, for example a ReLU activation layer, such as a leaky ReLU activation layer and a 3D-Pooling layer 614, for example a Max Pooling layer. By way of example, the 3D-convolution window of the 3D-convolutional layers 611 has a size of 3x3x3 voxels, and the window used by the 3D-Pooling layers 614 has a size of 2x2x2 voxels. In this example, the window sizes are the same for all convolutional neuromorphic processing layers, but this is not necessary. In the example shown the 3D-convolutional layer 611 of the first convolutional neuromorphic processing layer 6_1 has 8 filters, and the 3D-convolutional layer 611 of each subsequent convolutional neuromorphic processing layer has twice the number of filters of the preceding one. Therewith the convolutional neuromorphic processing unit 6 provides at its output a 128-dimensional feature vector.

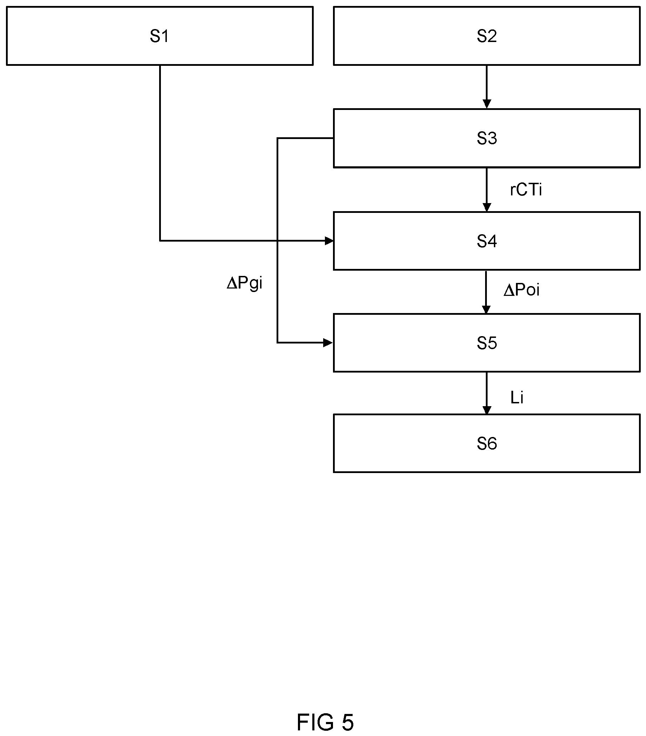

FIG. 5 schematically illustrates a method for training the neuromorphic dose based control module 41. As shown therein, the method comprises the following operations.

First volumetric image data, e.g. planning CT image data pCT of a predetermined planning CT image is provided in step S1 to the first convolutional neuromorphic processing branch 411.

Second volumetric image data, for example from a predetermined repeated CT image data is provided is step S2 and an augmented set of predetermined second image data {rCT1,...,rCTn} is obtained in step S3 by applying mutually different transformations {ΔP1,..., ΔPn} in three dimensional space to the predetermined repeated CT image rCT. In addition the Golden Truth {ΔPg1,..., ΔPgn} for the required correction for each species of the augmented set of second images is computed from the GT indication of the second image and the transformation applied to that species.

In other examples the volumetric image data is synthetic CT (sCT) image data, e.g. based on CBCT or MRI.

Each of the second image data {rCT1,...,rCTn} is then provided in step S4 to the convolutional neuromorphic processing branch 412.

As a result, the dose based control module 41 computes for each pair of first image data pCT and a species rCTi of the augmented set of second image data an output vector {ΔPo1,..., ΔPon} that is indicative for a correction in the registration.

In step S5 for each pair i a loss Li is computed from the difference between the correction specified by the output vector ΔPoi computed by the dose based control module 41 and the respective GT ΔPgi for that pair.

The weights of the first and the second, mutually identical convolutional neuromorphic processing branch 411, 412, the neuromorphic concatenation stage 413 and the fully connected neuromorphic stage 414 are then updated in step S6 to minimize the loss Li, for example using backpropagation.

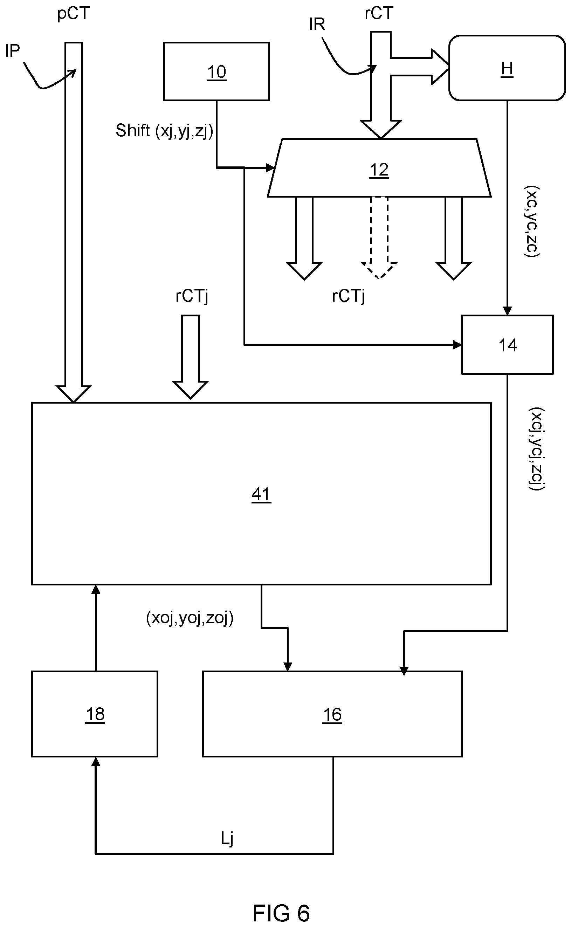

FIG. 6 schematically shows a training system for training the dose based control module 41. As shown in FIG. 6, the training system comprises a first input IP for receiving a first image of the patient, in this case a planning CT image pCT comprising tissue label data and dose specification data. The training system also comprises a second input IR for receiving a second image rCT of the patient. The second image rCT includes a designated part of the patient to be treated and is taken at a subsequent point in time. An oncologist H determines which correction, e.g. a position correction (xc, yc, zc) of the patient is necessary to optimally deliver the dose as originally intended by the treatment plan. A transformation vector generating unit 10 generates a plurality of transformations, e.g. translations (xi, yi, zi) to be applied to the second image **rCT** by an image transformation unit 12 to obtain transformed second images **rCTi.** The correction (xc, yc, zc) is taken as the Ground Truth (GT) value of the correction that is optimal for the second image **rCT.** Based on this GT value and the known transformation applied to the second image **rCT** to obtain the translated second images **rCTi,** the addition unit 14 computes the corresponding GT-value (xcj, ycj, zcj) for each transformed second image **rCTi.** The dose based control module 41 that is to be trained compares each translated second image **rCTj** with the first image pCT and estimates the required correction vector (xoj, yoj, zoj). The loss computation module 16 determines the difference between the estimated correction vector (xoj, yoj, zoj) and the corresponding GT-value (xcj, ycj, zcj) and outputs a Loss value Lj, for example a value indicative for a distance between the estimated correction vector (xoj, yoj, zoj) and the corresponding GT-value (xcj, ycj, zcj) in the space defining the translation vector. The distance measure is for example a Manhattan distance, an Euclidian distance or an L∝ measure. The current network parameters of the dose based control module 41 are then updated so as to minimize the loss, for example using back propagation.

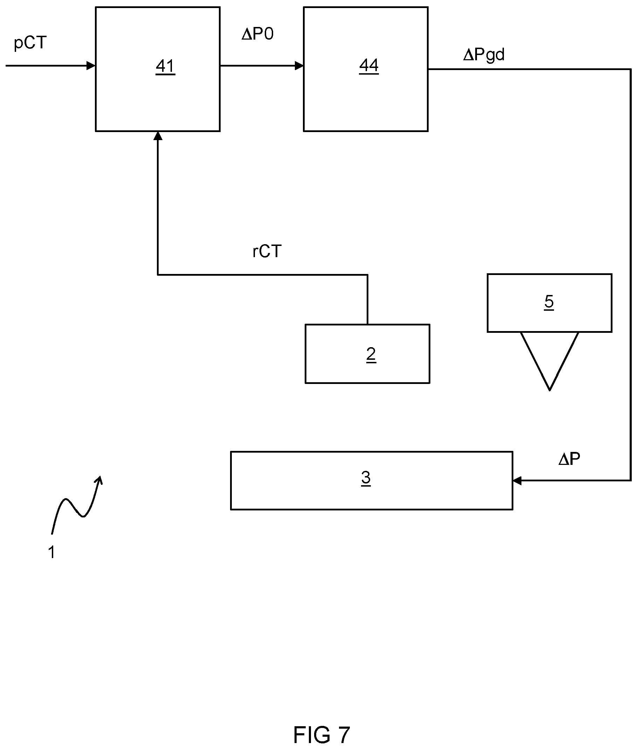

FIG. 7 shows a further embodiment of the positioning system that further comprises a third control module 44 configured to compute a complete dose-guided optimization by applying a gradient descent GD algorithm. The third control module 44 receives a output vector ΔP0 indicative for a correction in the registration as computed by the dose based control module 41. In an alternative embodiment the input of the third control module 44 is the output ΔP as computed in the embodiment shown in FIG. 2.

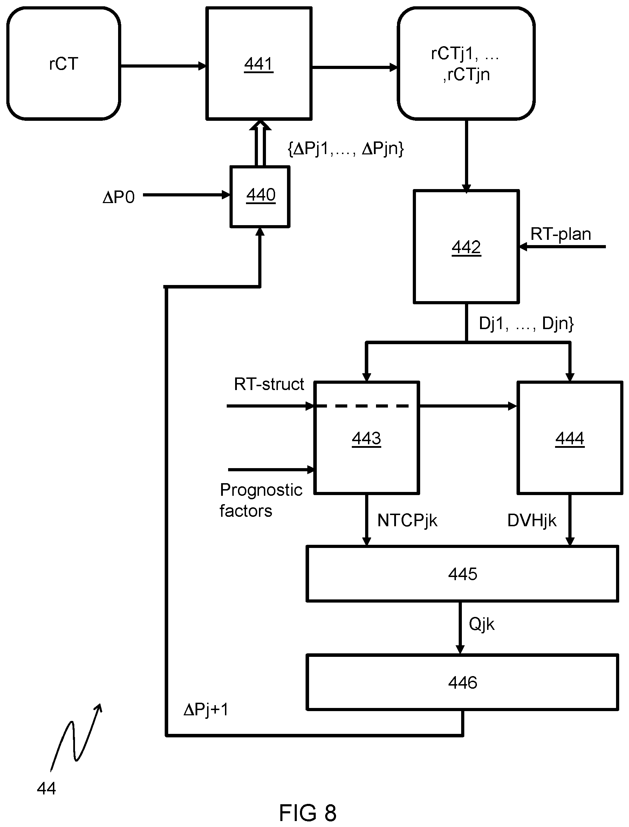

FIG. 8 shows an embodiment of the third control module 44 in more detail. As shown in that embodiment, a transformation vector modifier 440 is provided that generates a set of modified transformation vectors {ΔPj1,..., ΔPjn} for each transformation vector ΔPj at its input. Accordingly, for the transformation vector ΔP0 received from the dose based control module 41 it generates the set of modified transformation vectors {ΔP01,..., ΔP0n}. The image transformation module 441 provides a set of transformed second image data {rCTj1, ..., rCTjn}. Each species of modified second image data rCTjk is obtained by transforming the second image data (rCT) in accordance with a respective modified transformation vector ΔPjk.

Based on the predetermined radiation therapy plan (RT-plan), a dose simulation module 442 then provides for each modified repeated CT image data rCTjk an estimated spatial dose distribution Djk. The simulation module 442 for example uses a Monte Carlo simulation to estimate the spatial dose distribution Djk.

For each of the estimated spatial dose distributions Djk a treatment effect computation module 443 computes an indication NTCPjk of the effect of the estimated spatial dose distribution on the patient. This computation takes into account RT-structure information and information about prognostic factors.

In addition a dose volume histogram (444) computation module computes for each estimated spatial dose distributions Djk an expected dose volume histogram DVHjk taking into account RT-structure information.

An evaluation module 445 computes an overall quality measure Qjk that indicates the expected quality Qjk of the treatment based on the computed indications NTCPjk and DVHjk for the estimated spatial dose distributions Djk.

The gradient descent module 446 computes a single next iterated transformation vector ΔPj+1. To that end the gradient descent module 446 determines which direction in the space wherein the transformation vector is defined results in the highest improvement of the expected quality.

By way of example, in case the transformation vector ΔPj comprises the components (Δxj, Δyj, Δzj), the transformation vector modifier 440 may generate a set of modified transformation vectors {ΔPj1,..., ΔPjn}, wherein the modified transformation vector has components (Δxj+dxk, Δyj+dyk, Δzj+dzk), wherein the displacements dxk, dyk, dzk are uniformly distributed in a volume centered around the origin in the space of the transformation vector. The gradient descent module 446 may then select as the next iterated transformation vector ΔPj+1 the one of the modified transformation vectors ΔPjk corresponding to the highest value for Qjk.

In the embodiments described above, it is presumed that the transformation vector space is a three-dimensional space. The transformation vector in that case is a three-dimensional vector in the mutually orthogonal spatial directions x,y,z. In these embodiments the dose based control module (41) is configured to provide positioning control data ΔP a three-dimensional control vector that causes the positioning device to assume a corrected position in the three-dimensional space. In practice already substantial improvements are achieved therewith. In other embodiments the transformation vector is a higher dimensional vector that not only comprises components for the spatial directions x,y,z, but also comprises one or more components related to the orientation in space. In these other embodiments the dose based control module 41 is not only configured to provide positioning control data to cause the positioning device to assume a corrected position in the three-dimensional space, but the dose based control module 41 is also configured to provide orientation control data to cause the positioning device to assume a corrected orientation in the three-dimensional space. Therewith a further improvement of dose control is possible.

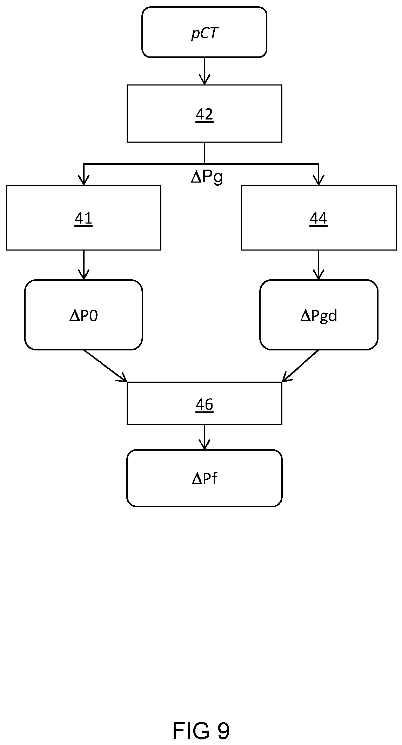

FIG. 9 schematically shows a further embodiment of the system. As in the embodiment of FIG. 2, a geometry based control module 42 is included. In this embodiment the geometry based control module 42 provides its computed correction vector ΔPg to the input of the dose based control module 42 as well as to the input of the further control module 44. Based on the correction vector ΔPg provided at their respective inputs the dose based control module 42 and the further control module 44 each compute a correction vector ΔP0, ΔPg respectively for dose based positioning. A quality assessment module 46 additionally comprised in this embodiment of the system compares the correction vector ΔPg computed by the further control module 44 with the correction vector ΔP0 computed by the dose based control module 42. Based on the comparison the quality assessment module 46 determines a final correction vector ΔPf, and the system positions the patient according to the final correction vector ΔPf so obtained.

### EXPERIMENTAL RESULTS

In a first experiment the neural network as shown in and described with reference to FIG. 3 was trained for the purpose of providing a geometry based correction vector.

A first test was performed with one patient image pair (pCT + rCT). The rCT-image was augmented 1000x by applying synthetic shifts between -10 and 10cm over the x, y and z directions.

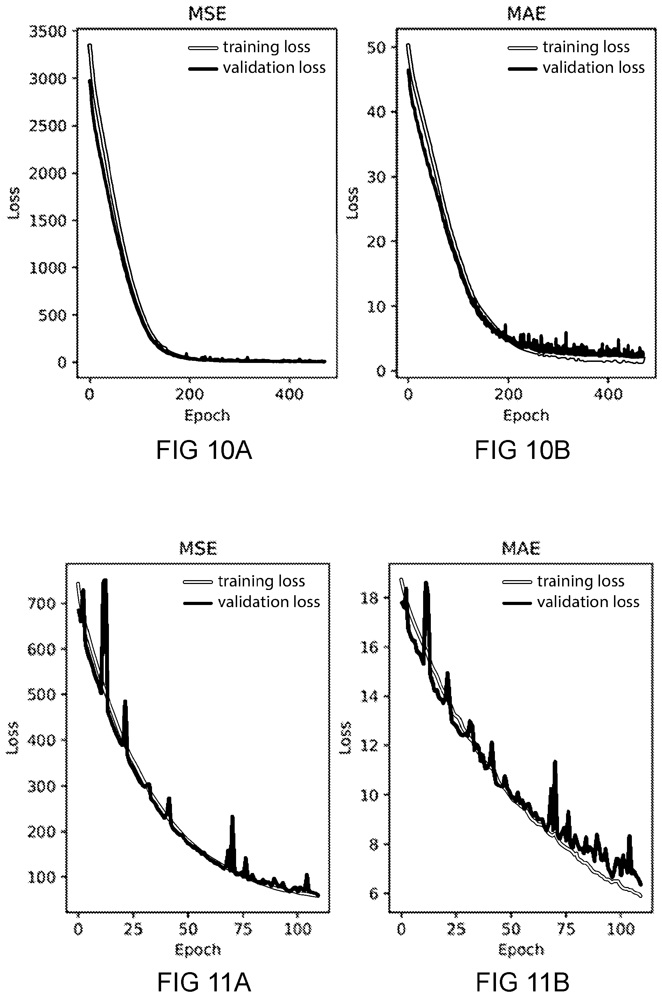

Results of the training process are shown in FIG. 10A and 10B.

FIG. 10A and 10B respectively show the training loss expressed as the mean square error (MSE) and the mean absolute error (MAE) as a function of the number of training epochs. Upon completion of the training the neural network estimated the geometrically based correction vector with a Mean Absolute Error of less than 1 mm for all axis.

In a second experiment a second training/validation set was used where data from 45 patients was used. Each patient had one pCT and 5 repeated CTs, for a total of 225 image pairs. These images were then augmented x5 with synthetic shifts of -5 to +5 cm in x, y and z axis. The total number of images used was 1125 images.

Results of the training process are shown in FIG. 11A - 11D.

FIG. 11A and 11B respectively show the training loss expressed as the mean square error (MSE) and the mean absolute error (MAE) as a function of the number of training epochs during a short training period (100 epochs).

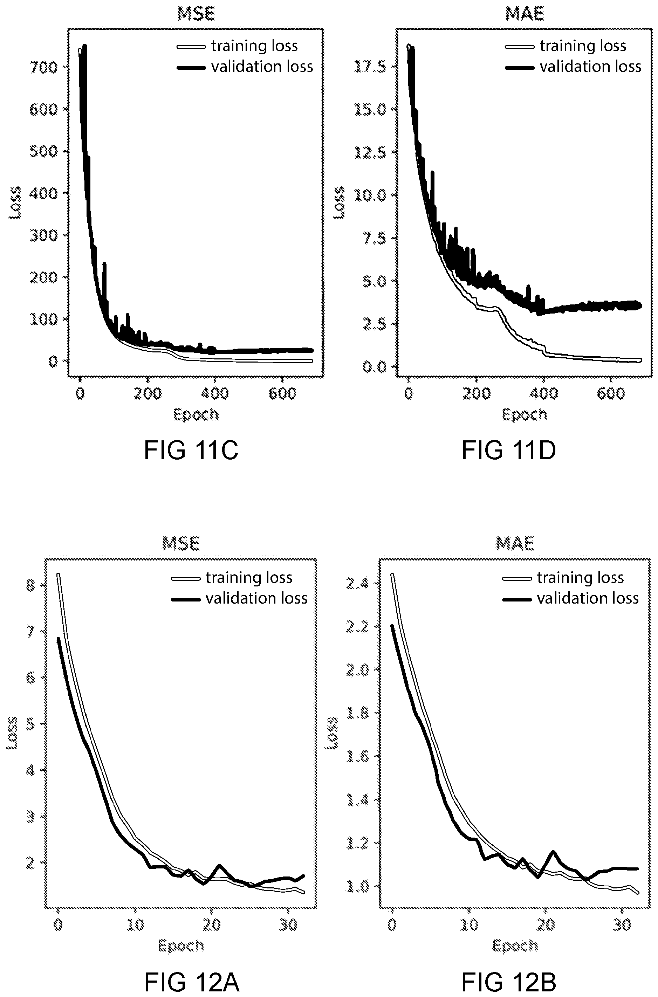

FIG. 11C and 11D respectively show the training loss expressed as the mean square error (MSE) and the mean absolute error (MAE) as a function of the number of training epochs during a long training period (600 epochs). Upon completion of the long training period the neural network estimated the geometrically based correction vector with a Mean Absolute Error of less than 3 mm for all axis.

In a third experiment the neural network as shown in and described with reference to FIG. 3 was trained for the purpose of providing a dose based correction vector. In this case, a daily dose is first evaluated at the current patient position in the rCT images. Then, the network is trained with the planning dose and daily dose in order to optimize a translational shift previously obtained by gradient descent.

Results of the training process are shown in FIG. 12A - 12D.

FIG. 12A and 12B respectively show the training loss expressed as the mean square error (MSE) and the mean absolute error (MAE) as a function of the number of training epochs during a short training period (30 epochs).

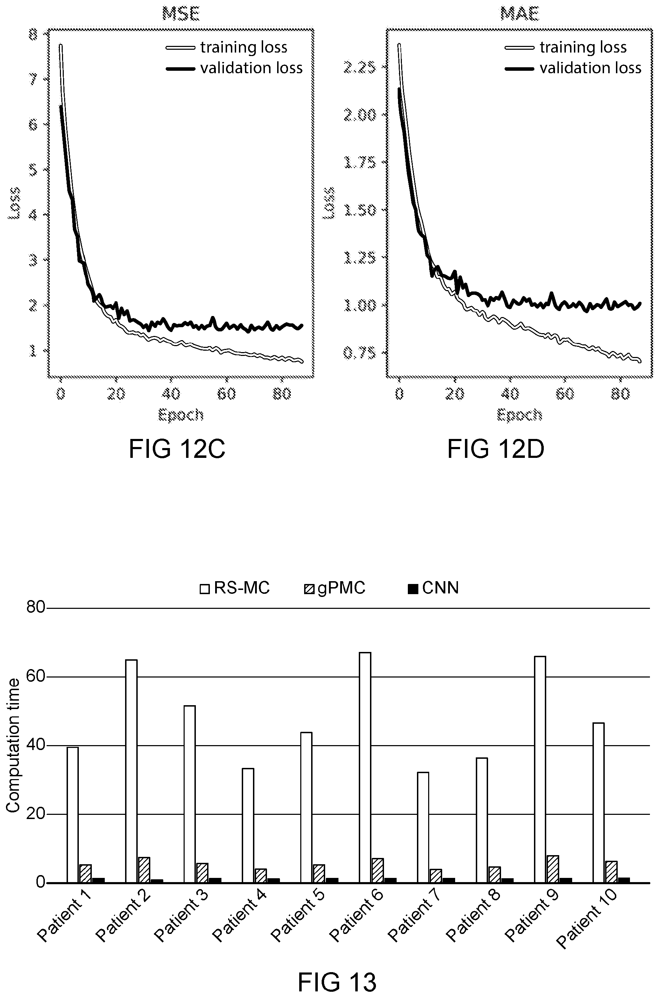

FIG. 12C and 12D respectively show the training loss expressed as the mean square error (MSE) and the mean absolute error (MAE) as a function of the number of training epochs during a long training period (80 epochs). Upon completion of the long training period the neural network estimated the dose based correction vector with a Mean Absolute Error of less than 1.2 mm for all axis. In any case a relatively modest number of training examples is sufficient for the network which is already trained to compute a geometry based correction as training does not need to start from scratch.

FIG. 13 shows a comparison of the computation speed involved in three approaches to achieve a dose-based optimization. The comparison was performed for 10 head and neck cancer patients previously treated with proton radiotherapy at the GPTC. For each patient, these computations require imaging data (planning CT and repeated CTs, segmentation files) and treatment data (radiotherapy plan) in DICOM format.

The three approaches comprise:
1) The RS-MC dose engine from RaySearch's Treatment Planning System (TPS) software called RayStation.
2) The gradient descent method when using the fast Monte Carlo code called gPMC, which has been developed at the Massachusetts General Hospital (MGH). This is a GPU based dose engine for proton therapy which focuses on dose and LET calculations only.
3) The computation by the CNN as specified with reference to FIG. 4.

For each patient the left column shows the computation time involved The computation according to approaches 1 and 2 were performed with the GPU-based clinical RayStation client configuration comprising:
∘ 12 physical cores dual socket Intel 2.6GHz CPUs
∘ NVIDIA RTX8000 GPU
∘ 48 GB DDR3 ECC RAM
∘ 2x 150 SSDs in RAID 1

The CNN based optimization was performed with the CNN of FIG. 4 implemented on the clinical QA platform server at the GPTC, comprising:
∘ 24 physical cores dual socket Intel 3.0GHz CPUs
∘ 2x NVIDIA GTX 1080 GPUs
∘ 64 GB DDR4 RAM
∘ 4x 250 GB SSDs

FIG. 13 shows for each patient the computation times involved with the approaches 1), 2) and 3) respectively.

The results are summarized in the following table

| Approach | Tmin (min) | Tmax (min) | Tav (min) |
|---|---|---|---|
| RS-MC | 32 | 65 | 45 |
| gMC | 4 | 8 | 6 |
| CNN | 1 | 1 | 1 |

In summary it can be seen that a very fast dose based optimization is achieved with the CNN-based method (3). Even if the CNN-based computation were performed with the GPU-based clinical RayStation client configuration it would still be significantly faster than the RS-MC based method. The optimization result obtained by this method (3) can be independently verified with the gMC based approach (2), which also is substantially faster than the RS-MC based approach. Therewith also the verified optimization result can be achieved substantially faster than is possible with the RS-MC based approach.

## Claims

1. A system (1) for positioning a patient for treatment in a beam of radiation in accordance with patient specific data including a first image (pCT) of the patient comprising tissue label data and dose specification data, the system comprising:
an imaging device (2) configured to provide a second image (rCT) of the patient including a designated part of the patient to be treated;
a positioning device (3) for holding the patient in a variable position and/or orientation in the beam of radiation for an accurate intervention to a designated part of the patient; and
an optimization controller (4) configured to provide registration control data (ΔP) to guide the positioning device (3) based on the first image (pCT) and the second image (rCT); wherein the optimization controller (4) comprises a dose based control module (41) configured to compute an optimized registration of the patient by performing a dose-guided optimization,
the dose based control module being configured to:
perform neuromorphic data processing operations to first image data obtained from said first image (pCT) to render first feature map data (F1);
perform said neuromorphic data processing operations to second image data obtained from said second image (rCT) to render second feature map data (F2);
concatenate the first feature map data (F1) and the second feature map data (F2) to provide concatenated feature map data (Fc);
perform a further neuromorphic data processing operations to the concatenated feature map data (Fc) to provide a correction vector indicative for a required registration of the patient for dose based optimization,
**characterized in that** the system comprises a further control module (44) configured to compute a correction vector for dose-guided optimization by applying a gradient descent (GD) algorithm,
the further control module (44) comprising:
a transformation vector modifier (440) to generate a set of modified transformation vectors ({ΔPj1,..., ΔPjn}) for each transformation vector (ΔPj) at its input;
an image transformation module (441) to provide a set of modified second images ({rCTj1, ..., rCTjn}) by transforming the second image (rCT) in accordance with respective modified transformation vectors (ΔPjk) of the set of modified transformation vectors ({ΔPj1,..., ΔPjn});
a dose simulation module (442) to provide for each modified second image (rCTjk) an estimated spatial dose distribution (Djk);
a dose volume histogram (444) computation module to compute an expected dose volume histogram (DVHjk) resulting for each estimated spatial dose distribution (Djk);
an evaluation module (445) to compute a quality measure (Qjk) that indicates the expected quality (Qjk) of the treatment based on the computed expected dose volume histogram (DVHjk) for each estimated spatial dose distribution (Djk);
a gradient descent module (446) to compute a single next iterated transformation vector (ΔPj+1) based on the respective values for the expected quality (Qjk) determined for the modified transformation vectors (ΔPjk) of the set of modified transformation vectors ({ΔPj1,..., ΔPjn}).

2. The system (1) according to claim 1, further comprising:
a geometry based control module (42) to perform a geometrical rigid registration to provide a first estimate (ΔPg) for a required registration correction of the patient based on the first image (pCT) and the second image (rCT),
a transformation module (43) to transform either of the first image or the second image to compensate for a difference in registration as indicated by the first estimate (ΔPg),
wherein the dose based control module (41) is configured to determine an estimate (ΔPd) for a required additional registration correction of the patient based on the first image (pCT) and the second image (rCT) of which one is transformed.

3. The system according to claim 1 or 2, wherein the dose based control module (41) comprises:
a first convolutional neuromorphic processing branch (411) being configured to receive the first image data obtained from the first image (pCT) and to provide the first feature map data (F1) based on said received first image data,
a second convolutional neuromorphic processing branch (412) identical to the first convolutional neuromorphic processing branch (411) and being configured to receive the second image data obtained from the second image (rCT) and to provide the second feature map data (F2) based on said received second image data,
- a neuromorphic concatenation stage (413) being configured to receive the first feature map data (F1) and the second feature map data (F2) and to provide the concatenated feature map data (Fc) based on the first feature map data and the second feature map data;
- a fully connected neuromorphic stage (414) being configured to receive the concatenated feature map data (Fc) and to provide the correction vector (ΔPd) indicative for the correction in the registration.

4. The system according to claim 3, wherein the mutually identical convolutional neuromorphic processing branches (411, 412) each comprise a plurality of stages (6_1,...,6_n) with a convolutional neuromorphic layer (611).

5. The system according to claim 4, wherein each stage (6_1,...,6_n) of the convolutional neuromorphic processing branches comprises a batch normalization layer (612).

6. The system according to claim 4 or 5, wherein each stage (6_1,...,6_n) of the convolutional neuromorphic processing branches comprises a leaky ReLU activation layer (613).

7. The system according to claim 4, 5 or 6, wherein each stage (6_1,...,6_n) of the convolutional neuromorphic processing branches comprises a pooling layer (614).

8. The system according to claim 5, wherein the pooling layer (614) is a max pooling layer.

9. The system according to one of the preceding claims , wherein the simulation module (442) estimates the spatial dose distribution (Djk) with a Monte Carlo simulation.

10. The system according to any of the preceding claims, further comprising a radiation therapy, RT, treatment effect computation module (443) to provide an estimation of the normal tissue complication probability (NTCPjk) associated with each estimated spatial dose distribution (Djk) on the human body, taking into account one or more of RT-structure information and information about prognostic factors wherein the evaluation module (445) is configured to compute the quality measure (Qjk) based on the computed expected dose volume histogram (DVHjk) and based on the estimation of said probability.

11. The system according to any of the preceding claims, being configured to update parameters of the dose based control module (41) based on a loss defined by a difference between the correction vector determined by the third control module (44) and the correction vector determined by the dose based control module (41).

12. The system according to any of the preceding claims, wherein the further control module (44) is configured to receive the correction vector (ΔP0) provided by the dose based control module (41) at its input and wherein the system is configured to position the patient according to the correction vector computed by the further control module.

13. The system according to claim 2, wherein the further control module (44) is configured to receive the correction vector (ΔPg) provided by the geometry based control module (42) at its input, the system further comprising a quality assessment module (46) to compare the correction vector computed by the further control module (44) with the correction vector computed by the dose based control module (42), wherein the system is configured to position the patient according to a correction vector (ΔPf) determined by the quality assessment module (46) based on the comparison.

14. A training system for training a dose based control module (41) comprising:
a first input (IP) for receiving a first image (pCT) of a patient, the first image comprising tissue label data and dose specification data;
a second input (IR) for receiving a second image (rCT) of the patient, the second image (rCT) including a designated part of the patient to be treated and being taken at a point in time later than the first image;
a third input (H) to receive a Ground Truth, GT, indication of a transformation vector specifying a correction (xc, yc, zc) of the position and/or orientation of the patient with which a dose delivered to the patient optimally approximates the dose as originally intended in accordance with the first image;
a transformation vector generator unit (10) to generate a plurality of transformation vectors (xi, yi, zi);
an image transformation unit (12) to obtain respective transformed second images (rCTi) by applying the transformation vectors (xi, yi, zi) to the second image (rCT);
an addition unit (14) which for each transformed second image (rCTi) computes the corresponding GT-correction vector (xcj, ycj, zcj) from the GT-indication of the second image (rCT) and the transformation vector (xi, yi, zi) with which the transformed second image (rCTi) was obtained;
a loss computation module (16) to determine for each transformed second image a loss based on a difference between the corresponding GT-correction vector and the correction vector (xoj, yoj, zoj) estimated by the dose based control module when the first image and the transformed second image are provided as input;
an update module (18) for updating parameters of the dose based control module (41) based on the computed loss.

15. A method for training a neuromorphic dose based control module (41), comprising:
Providing (S1) a first image (pCT) of a patient comprising tissue label data and dose specification data;
Providing (S2) a second image (rCT) of the patient including a designated part of the patient to be treated, therewith also providing a Ground Truth, GT, indication of a transformation vector specifying a correction (xc, yc, zc) of the position and/or orientation of the patient with which a dose delivered to the patient optimally approximates the dose as originally intended in accordance with the first image;
Generating (S3) an augmented set of second images ({rCT1,...,rCTn}) by applying mutually different geometrical transformation {ΔP1,..., ΔPn} in three dimensional space to the second image (rCT) and for each species of the augmented set of second images computing the Ground Truth for the required correction from the GT indication of the second image and the transformation applied to that species;
With the dose based control module (41) computing (S4) a respective output vector {ΔPo1,..., ΔPon} indicative for a correction (xc, yc, zc) of the position and/or orientation of the patient for each pair of the first image (pCT) and a species (rCTi) of the augmented set of second images ({rCT1,...,rCTn});
For each species (rCTi) of the augmented set of second images ({rCT1,...,rCTn}) computing (S5) a loss (Li) from the difference between the correction specified by the respective output vector and the respective GT; Updating (S6) parameters of the dose based control module (41) to reduce the loss (Li).

## Patentansprüche

1. System (1) zum Positionieren eines Patienten für eine Behandlung in einem Strahlenbündel in Übereinstimmung mit patientenspezifischen Daten, die ein erstes Bild (pCT) des Patienten einschließen, das Gewebemarkierungsdaten und Dosisspezifikationsdaten einschließen, wobei das System Folgendes umfasst:
eine Bildgebungsvorrichtung (2), die dazu konfiguriert ist, ein zweites Bild (rCT) des Patienten bereitzustellen, das einen bestimmten Teil des zu behandelnden Patienten einschließt;
eine Positionierungsvorrichtung (3) zum Halten des Patienten in einer variablen Position und/oder Ausrichtung in dem Strahlenbündel für eine präzise Intervention an einem bestimmten Teil des Patienten; und
eine Optimierungssteuerung (4), die dazu konfiguriert ist, Registrierungssteuerungsdaten (ΔP) zum Führen der Positionierungsvorrichtung (3) basierend auf dem ersten Bild (pCT) und dem zweiten Bild (rCT) bereitzustellen; wobei die Optimierungssteuerung (4) ein dosisbasiertes Steuermodul (41) umfasst, das dazu konfiguriert ist, eine optimierte Registrierung des Patienten durch Ausführen einer dosisgesteuerten Optimierung zu berechnen,
wobei das dosisbasierte Steuermodul zu Folgendem konfiguriert ist:
Ausführen neuromorpher Datenverarbeitungsoperationen an ersten Bilddaten, die aus dem ersten Bild (pCT) gewonnen wurden, um erste Merkmalkartendaten (F1) zu erzeugen;
Ausführen der neuromorphen Datenverarbeitungsoperationen an zweiten Bilddaten, die aus dem zweiten Bild (rCT) gewonnen wurden, um zweite Merkmalkartendaten (F2) zu erzeugen;
Verknüpfen der ersten Merkmalkartendaten (F1) und der zweiten Merkmalkartendaten (F2), um verknüpfte Merkmalkartendaten (Fc) bereitzustellen;
Ausführen einer weiteren neuromorphen Datenverarbeitungsoperation an den verketteten Merkmalkartendaten (Fc), um einen Korrekturvektor bereitzustellen, der indikativ für eine erforderliche Registrierung des Patienten für eine dosisbasierte Optimierung ist,
**dadurch gekennzeichnet, dass** das System ein weiteres Steuermodul (44) umfasst, das dazu konfiguriert ist, einen Korrekturvektor für eine dosisgeführte Optimierung durch Anwenden eines Gradientenabstiegsalgorithmus (GD-Algorithmus) zu berechnen,
wobei das weitere Steuermodul (44) Folgendes umfasst:
einen Transformationsvektormodifikator (440) zum Erzeugen eines Satzes modifizierter Transformationsvektoren ({ΔPj1, ..., ΔPjn}) für jeden Transformationsvektor (ΔPj) an seinem Eingang;
ein Bildtransformationsmodul (441) zum Bereitstellen eines Satzes modifizierter zweiter Bilder ({rCTj 1, ..., rCTjn}) durch Transformieren des zweiten Bildes (rCT) in Übereinstimmung mit den jeweiligen modifizierten Transformationsvektoren (ΔPjk) des Satzes modifizierter Transformationsvektoren ({ΔPj1, ..., ΔPjn});
ein Dosissimulationsmodul (442) zum Bereitstellen einer geschätzten räumlichen Dosisverteilung (Djk) für jedes modifizierte zweite Bild (rCTjk);
ein Berechnungsmodul für Dosis-Volumen-Histogramme (444) zum Berechnen eines erwarteten Dosis-Volumen-Histogramms (DVHjk), das sich für jede geschätzte räumliche Dosisverteilung (Djk) ergibt;
ein Auswertungsmodul (445) zum Berechnen eines Qualitätsmaßes (Qjk), das die erwartete Qualität (Qjk) der Behandlung basierend auf dem berechneten erwarteten Dosis-Volumen-Histogramm (DVHjk) für jede geschätzte räumliche Dosisverteilung (Djk) angibt;
ein Gradientenabstiegsmodul (446) zum Berechnen eines einzelnen nächsten iterierten Transformationsvektors (ΔPj+1) basierend auf den jeweiligen Werten für die erwartete Qualität (Qjk), die für die modifizierten Transformationsvektoren (ΔPjk) des Satzes modifizierter Transformationsvektoren ({ΔPj1, ..., ΔPjn}) bestimmt wurden.

2. System (1) nach Anspruch 1, ferner umfassend:
ein geometriebasiertes Steuermodul (42) zum Ausführen einer geometrischen starren Registrierung, um eine erste Schätzung (ΔPg) für eine erforderliche Registrierungskorrektur des Patienten basierend auf dem ersten Bild (pCT) und dem zweiten Bild (rCT) bereitzustellen,
ein Transformationsmodul (43) zum Transformieren entweder des ersten Bildes oder des zweiten Bildes, um eine durch die erste Schätzung (ΔPg) angegebene Differenz in der Registrierung zu kompensieren,
wobei das dosisbasierte Steuermodul (41) dazu konfiguriert ist, eine Schätzung (ΔPd) für eine erforderliche zusätzliche Registrierungskorrektur des Patienten basierend auf dem ersten Bild (pCT) und dem zweiten Bild (rCT) zu bestimmen, von denen eines transformiert wird.

3. System nach Anspruch 1 oder 2, wobei das dosisbasierte Steuermodul (41) Folgendes umfasst:
einen ersten konvolutionellen neuromorphen Verarbeitungszweig (411), der dazu konfiguriert ist, die aus dem ersten Bild (pCT) erhaltenen ersten Bilddaten zu empfangen und basierend auf den empfangenen ersten Bilddaten die ersten Merkmalkartendaten (F1) bereitzustellen,
einen zweiten konvolutionellen neuromorphen Verarbeitungszweig (412), der mit dem ersten konvolutionellen neuromorphen Verarbeitungszweig (411) identisch ist und dazu konfiguriert ist, die aus dem zweiten Bild (rCT) erhaltenen zweiten Bilddaten zu empfangen und basierend auf den empfangenen zweiten Bilddaten die zweiten Merkmalkartendaten (F2) bereitzustellen,
- eine neuromorphe Verknüpfungsstufe (413), die dazu konfiguriert ist, die ersten Merkmalkartendaten (F1) und die zweiten Merkmalkartendaten (F2) zu empfangen und die verknüpften Merkmalkartendaten (Fc) basierend auf den ersten Merkmalkartendaten und den zweiten Merkmalkartendaten bereitzustellen;
- eine vollständig verbundene neuromorphe Stufe (414), die dazu konfiguriert ist, die verknüpften Merkmalkartendaten (Fc) zu empfangen und den Korrekturvektor (APd) bereitzustellen, der indikativ für die Korrektur bei der Registrierung ist.

4. System nach Anspruch 3, wobei die miteinander identischen konvolutionellen neuromorphen Verarbeitungszweige (411, 412) jeweils eine Vielzahl von Stufen (6_1, ..., 6_n) mit einer konvolutionellen neuromorphen Schicht (611) umfassen.

5. System nach Anspruch 4, wobei jede Stufe (6_1,...,6_n) der konvolutionellen neuromorphen Verarbeitungszweige eine Batch-Normalisierungsschicht (612) umfasst.

6. System nach Anspruch 4 oder 5, wobei jede Stufe (6_1,...,6_n) der konvolutionellen neuromorphen Verarbeitungszweige eine leaky-ReLU-Aktivierungsschicht (613) umfasst.

7. System nach Anspruch 4, 5 oder 6, wobei jede Stufe (6_1, ..., 6_n) der konvolutionellen neuromorphen Verarbeitungszweige eine Pooling-Schicht (614) umfasst.

8. System nach Anspruch 5, wobei die Pooling-Schicht (614) eine Max-Pooling-Schicht ist.

9. System nach einem der vorstehenden Ansprüche, wobei das Simulationsmodul (442) die räumliche Dosisverteilung (Djk) mit einer Monte-Carlo-Simulation schätzt.

10. System nach einem der vorstehenden Ansprüche, ferner umfassend ein Behandlungswirkungsberechnungsmodul (443) für Strahlentherapie, RT, um eine Schätzung der Wahrscheinlichkeit von Komplikationen in normalem Gewebe (NTCPjk) bereitzustellen, die jeder geschätzten räumlichen Dosisverteilung (Djk) auf den menschlichen Körper zugeordnet ist, unter Berücksichtigung einer oder mehrerer RT-Strukturinformationen und Informationen über Prognosefaktoren, wobei das Auswertungsmodul (445) dazu konfiguriert ist, das Qualitätsmaß (Qjk) basierend auf dem berechneten erwarteten Dosis-Volumen-Histogramm (DVHjk) und basierend auf der Schätzung der Wahrscheinlichkeit zu berechnen.

11. System nach einem der vorstehenden Ansprüche, das dazu konfiguriert ist, Parameter des dosisbasierten Steuermoduls (41) basierend auf einem Verlust zu aktualisieren, der durch eine Differenz zwischen dem von dem dritten Steuermodul (44) bestimmten Korrekturvektor und dem von dem dosisbasierten Steuermodul (41) bestimmten Korrekturvektor definiert ist.

12. System nach einem der vorstehenden Ansprüche, wobei das weitere Steuermodul (44) dazu konfiguriert ist, den von dem dosisbasierten Steuermodul (41) bereitgestellten Korrekturvektor (ΔP0) an seinem Eingang zu empfangen, und wobei das System dazu konfiguriert ist, den Patienten gemäß dem von dem weiteren Steuermodul berechneten Korrekturvektor zu positionieren.

13. System nach Anspruch 2, wobei das weitere Steuermodul (44) dazu konfiguriert ist, den von dem geometriebasierten Steuermodul (42) bereitgestellten Korrekturvektor (ΔPg) an seinem Eingang zu empfangen, wobei das System ferner ein Qualitätsbewertungsmodul (46) umfasst, um den von dem weiteren Steuermodul (44) berechneten Korrekturvektor mit dem von dem dosisbasierten Steuermodul (42) berechneten Korrekturvektor zu vergleichen, wobei das System dazu konfiguriert ist, den Patienten gemäß einem Korrekturvektor (ΔPf) zu positionieren, der von dem Qualitätsbewertungsmodul (46) basierend auf dem Vergleich bestimmt wird.

14. Trainingssystem zum Trainieren eines dosisbasierten Steuermoduls (41), umfassend:
einen ersten Eingang (IP) zum Empfangen eines ersten Bildes (pCT) eines Patienten, wobei das erste Bild Gewebemarkierungsdaten und Dosisspezifikationsdaten umfasst;
einen zweiten Eingang (IR) zum Empfangen eines zweiten Bildes (rCT) des Patienten, wobei das zweite Bild (rCT) einen bestimmten Teil des zu behandelnden Patienten einschließt und zu einem späteren Zeitpunkt als das erste Bild aufgenommen wird;
einen dritten Eingang (H) zum Empfangen einer Ground-Truth-Angabe, GT-Angabe, eines Transformationsvektors, der eine Korrektur (xc, yc, zc) der Position und/oder Ausrichtung des Patienten angibt, mit der eine dem Patienten verabreichte Dosis die ursprünglich gemäß dem ersten Bild beabsichtigte Dosis optimal annähert;
eine Transformationsvektor-Generatoreinheit (10) zum Erzeugen einer Vielzahl von Transformationsvektoren (xi, yi, zi);
eine Bildtransformationseinheit (12) zum Erhalten entsprechender transformierter zweiter Bilder (rCTi) durch Anwenden der Transformationsvektoren (xi, yi, zi) auf das zweite Bild (rCT);
eine Additionseinheit (14), die für jedes transformierte zweite Bild (rCTi) den entsprechenden GT-Korrekturvektor (xcj, ycj, zcj) aus der GT-Angabe des zweiten Bildes (rCT) und dem Transformationsvektor (xi, yi, zi), mit dem das transformierte zweite Bild (rCTi) erhalten wurde, berechnet;
ein Verlustberechnungsmodul (16) zum Bestimmen eines Verlusts für jedes transformierte zweite Bild basierend auf einer Differenz zwischen dem entsprechenden GT-Korrekturvektor und dem von dem dosisbasierten Steuermodul geschätzten Korrekturvektor (xoj, yoj, zoj), wenn das erste Bild und das transformierte zweite Bild als Eingabe bereitgestellt werden;
ein Aktualisierungsmodul (18) zum Aktualisieren von Parametern des dosisbasierten Steuermoduls (41) basierend auf dem berechneten Verlust.

15. Verfahren zum Trainieren eines neuromorphen dosisbasierten Steuermoduls (41), umfassend:
Bereitstellen (S1) eines ersten Bildes (pCT) eines Patienten, das Gewebemarkierungsdaten und Dosisspezifikationsdaten umfasst;
Bereitstellen (S2) eines zweiten Bildes (rCT) des Patienten, das einen bestimmten Teil des zu behandelnden Patienten einschließt, wobei gleichzeitig eine Ground-Truth-Angabe, GT-Angabe, eines Transformationsvektors bereitgestellt wird, der eine Korrektur (xc, yc, zc) der Position und/oder Ausrichtung des Patienten angibt, mit der eine dem Patienten verabreichte Dosis die ursprünglich gemäß dem ersten Bild beabsichtigte Dosis optimal annähert;
Erzeugen (S3) eines erweiterten Satzes von zweiten Bildern ({rCT1, ..., rCTn}) durch Anwenden gegenseitig unterschiedlicher geometrischer Transformationen {ΔP1, ..., ΔPn} in dreidimensionalem Raum auf das zweite Bild (rCT) und für jede Variante des erweiterten Satzes von zweiten Bildern Berechnen der Ground Truth für die erforderliche Korrektur aus der GT-Angabe des zweiten Bildes und der auf diese Variante angewendeten Transformation;
mit dem dosisbasierten Steuermodul (41) Berechnen (S4) eines jeweiligen Ausgabevektors {ΔPo1, ..., ΔPon}, der indikativ für eine Korrektur (xc, yc, zc) der Position und/oder Ausrichtung des Patienten für jedes Paar des ersten Bildes (pCT) und einer Variante (rCTi) des erweiterten Satzes von zweiten Bildern ({rCT1, ..., rCTn}) ist;
für jede Variante (rCTi) des erweiterten Satzes von zweiten Bildern ({rCT1, ..., rCTn}) Berechnen (S5) eines Verlusts (Li) aus der Differenz zwischen der durch den jeweiligen Ausgabevektor angegebenen Korrektur und der jeweiligen GT;
Aktualisieren (S6) von Parametern des dosisbasierten Steuermoduls (41), um den Verlust (Li) zu reduzieren.

## Revendications

1. Système (1) pour positionner un patient sous un faisceau de rayonnement en vue d'un traitement conformément à des données spécifiques au patient comportant une première image (pCT) du patient comprenant des données de marquage de tissu et des données de spécification de dose, le système comprenant :
un dispositif d'imagerie (2) configuré pour fournir une deuxième image (rCT) du patient comportant une partie désignée du corps du patient à traiter ;
un dispositif de positionnement (3) pour maintenir le patient dans une position et/ou une orientation variables sous le faisceau de rayonnement pour une intervention précise sur une partie désignée du corps du patient ; et
un dispositif de commande d'optimisation (4) configuré pour fournir des données de commande d'enregistrement (ΔP) afin de guider le dispositif de positionnement (3) sur la base de la première image (pCT) et de la deuxième image (rCT) ; dans lequel le dispositif de commande d'optimisation (4) comprend un module de commande basé sur la dose (41) configuré pour calculer un enregistrement optimisé du patient en effectuant une optimisation guidée par dose,
le module de commande basé sur la dose étant configuré pour :
effectuer des opérations de traitement de données neuromorphiques sur des premières données d'image obtenues à partir de ladite première image (pCT) pour rendre des premières données de carte de caractéristiques (F1) ;
effectuer lesdites opérations de traitement de données neuromorphiques sur des deuxièmes données d'image obtenues à partir de ladite deuxième image (rCT) pour rendre des deuxièmes données de carte de caractéristiques (F2) ;
concaténer les premières données de carte de caractéristiques (F1) et les deuxièmes données de carte de caractéristiques (F2) pour fournir des données de carte de caractéristiques concaténées (Fc) ;
effectuer des opérations de traitement de données neuromorphiques supplémentaires sur les données de carte de caractéristiques concaténées (Fc) pour fournir un vecteur de correction indiquant un enregistrement requis du patient pour une optimisation basée sur la dose,
**caractérisé en ce que** le système comprend un module de commande supplémentaire (44) configuré pour calculer un vecteur de correction pour une optimisation guidée par dose en appliquant un algorithme de descente de gradient (GD),
le module de commande supplémentaire (44) comprenant :
un modificateur de vecteur de transformation (440) pour générer un ensemble de vecteurs de transformation modifiés ({ΔPj1, ..., ΔPjn}) pour chaque vecteur de transformation (ΔPj) à son entrée ;
un module de transformation d'images (441) pour fournir un ensemble de deuxièmes images modifiées ({rCTj1, rCTjn}) en transformant la deuxième image (rCT) conformément à des vecteurs de transformation modifiés (ΔPjk) respectifs de l'ensemble de vecteurs de transformation modifiés ({ΔPj1, ..., ΔPjn}) ;
un module de simulation (442) de dose pour fournir pour chaque deuxième image modifiée (rCTjk) une distribution de dose spatiale estimée (Djk) ;
un module de calcul d'histogramme dose-volume (444) pour calculer un histogramme dose-volume attendu (DVHjk) résultant pour chaque distribution de dose spatiale estimée (Djk) ;
un module d'évaluation (445) pour calculer une mesure de qualité (Qjk) qui indique la qualité (Qjk) attendue du traitement sur la base de l'histogramme dose-volume attendu (DVHjk) calculé pour chaque distribution de dose spatiale estimée (Djk) ;
un module de descente de gradient (446) pour calculer un seul vecteur de transformation itéré suivant (ΔPj+1) sur la base des valeurs respectives de la qualité (Qjk) attendue déterminées pour les vecteurs de transformation modifiés (ΔPjk) de l'ensemble de vecteurs de transformation modifiés ({ΔPj1, ..., ΔPjn}).

2. Système (1) selon la revendication 1, comprenant en outre :
un module de commande basé sur la géométrie (42) pour effectuer un enregistrement géométrique rigide afin de fournir une première estimation (ΔPg) pour une correction d'enregistrement requise du patient sur la base de la première image (pCT) et de la deuxième image (rCT),
un module de transformation (43) pour transformer soit la première image soit la deuxième image afin de compenser une différence d'enregistrement telle qu'indiquée par la première estimation (ΔPg),
dans lequel le module de commande basé sur la dose (41) est configuré pour déterminer une estimation (ΔPd) pour une correction d'enregistrement additionnelle requise du patient sur la base de la première image (pCT) et de la deuxième image (rCT) dont l'une est transformée.

3. Système selon la revendication 1 ou 2, dans lequel le module de commande basé sur la dose (41) comprend :
une première branche de traitement neuromorphique convolutif (411) configurée pour recevoir les premières données d'image obtenues à partir de la première image (pCT) et pour fournir les premières données de carte de caractéristiques (F1) sur la base desdites premières données d'image reçues,
une deuxième branche de traitement neuromorphique convolutif (412) identique à la première branche de traitement neuromorphique convolutif (411) et configurée pour recevoir les deuxièmes données d'image obtenues à partir de la deuxième image (rCT) et pour fournir les deuxièmes données de carte de caractéristiques (F2) sur la base desdites deuxièmes données d'image reçues,
- un stade de concaténation neuromorphique (413) configuré pour recevoir les premières données de carte de caractéristiques (F1) et les deuxièmes données de carte de caractéristiques (F2) et pour fournir les données de carte de caractéristiques concaténées (Fc) sur la base des premières données de carte de caractéristiques et des deuxièmes données de carte de caractéristiques ;
- un stade neuromorphique entièrement connecté (414) configuré pour recevoir les données de carte de caractéristiques concaténées (Fc) et pour fournir le vecteur de correction (ΔPd) indiquant la correction dans l'enregistrement.

4. Système selon la revendication 3, dans lequel les branches de traitement neuromorphique convolutif (411, 412) mutuellement identiques comprennent chacune une pluralité de stades (6_1, ..., 6_n) avec une couche neuromorphique convolutive (611).

5. Système selon la revendication 4, dans lequel chaque stade (6_1, ..., 6_n) des branches de traitement neuromorphique convolutif comprend une couche de normalisation par lots (612).

6. Système selon la revendication 4 ou 5, dans lequel chaque stade (6_1, ..., 6_n) des branches de traitement neuromorphique convolutif comprend une couche d'activation ReLU (613) de fuite.

7. Système selon la revendication 4, 5 ou 6, dans lequel chaque stade (6_1, ..., 6_n) des branches de traitement neuromorphique convolutif comprend une couche de regroupement (614).

8. Système selon la revendication 5, dans lequel la couche de regroupement (614) est une couche de regroupement maximal.

9. Système selon l'une des revendications précédentes, dans lequel le module de simulation (442) estime la distribution de dose spatiale (Djk) à l'aide d'une simulation Monte Carlo.

10. Système selon l'une des revendications précédentes, comprenant en outre un module de calcul d'effet de traitement par radiothérapie, RT, (443) pour fournir une estimation de la probabilité de complication de tissus sains (NTCPjk) associée à chaque distribution de dose spatiale estimée (Djk) sur le corps humain, en tenant compte d'informations de structure RT et/ou d'informations sur les facteurs pronostiques, dans lequel le module d'évaluation (445) est configuré pour calculer la mesure de qualité (Qjk) sur la base de l'histogramme dose-volume attendu (DVHjk) calculé et sur la base de l'estimation de ladite probabilité.

11. Système selon l'une des revendications précédentes, configuré pour mettre à jour des paramètres du module de commande basé sur la dose (41) sur la base d'une perte définie par une différence entre le vecteur de correction déterminé par le troisième module de commande (44) et le vecteur de correction déterminé par le module de commande basé sur la dose (41).

12. Système selon l'une des revendications précédentes, dans lequel le module de commande supplémentaire (44) est configuré pour recevoir le vecteur de correction (ΔP0) fourni par le module de commande basé sur la dose (41) à son entrée, et dans lequel le système est configuré pour positionner le patient selon le vecteur de correction calculé par le module de commande supplémentaire.

13. Système selon la revendication 2, dans lequel le module de commande supplémentaire (44) est configuré pour recevoir le vecteur de correction (ΔPg) fourni par le module de commande basé sur la géométrie (42) à son entrée, le système comprenant en outre un module d'évaluation de la qualité (46) pour comparer le vecteur de correction calculé par le module de commande supplémentaire (44) avec le vecteur de correction calculé par le module de commande basé sur la dose (42), dans lequel le système est configuré pour positionner le patient selon un vecteur de correction (ΔPf) déterminé par le module d'évaluation de la qualité (46) sur la base de la comparaison.

14. Système d'entraînement pour entraîner un module de commande basé sur la dose (41) comprenant :
une première entrée (IP) pour recevoir une première image (pCT) d'un patient, la première image comprenant des données de marquage de tissu et des données de spécification de dose ;
une deuxième entrée (IR) pour recevoir une deuxième image (rCT) du patient, la deuxième image (rCT) comportant une partie désignée du corps du patient à traiter et étant prise à un moment postérieur à la première image ;
une troisième entrée (H) pour recevoir une indication de réalité de terrain, GT, d'un vecteur de transformation spécifiant une correction (xc, yc, zc) de la position et/ou de l'orientation du patient permettant de rapprocher de manière optimale une dose administrée au patient de la dose initialement prévue conformément à la première image ;
une unité génératrice de vecteurs de transformation (10) pour générer une pluralité de vecteurs de correction (xi, yi, zi) ;
une unité de transformation d'images (12) pour obtenir des deuxièmes images transformées (rCTi) respectives en appliquant les vecteurs de correction (xi, yi, zi) à la deuxième image (rCT) ;
une unité d'addition (14) qui, pour chaque deuxième image transformée (rCTi), calcule le vecteur de correction GT (xcj, ycj, zcj) correspondant à partir de l'indication GT de la deuxième image (rCT) et du vecteur de transformation (xi, yi, zi) par lequel la deuxième image transformée (rCTi) a été obtenue ;
un module de calcul de perte (16) pour déterminer une perte pour chaque deuxième image transformée sur la base d'une différence entre le vecteur de correction GT correspondant et le vecteur de correction (xoj, yoj, zoj) estimés par le module de commande basé sur la dose lorsque la première image et la deuxième image transformées sont fournies comme entrée ;
un module de mise à jour (18) pour mettre à jour des paramètres du module de commande basé sur la dose (41) sur la base de la perte calculée.

15. Procédé pour entraîner un module de commande basé sur la dose (41) neuromorphique, comprenant les étapes suivantes :
fournir (S1) une première image (pCT) d'un patient comprenant des données de marquage de tissu et des données de spécification de dose ;
fournir (S2) une deuxième image (rCT) du patient comportant une partie désignée du corps du patient à traiter, fournir également avec cela une indication de réalité de terrain, GT, d'un vecteur de transformation spécifiant une correction (xc, yc, zc) de la position et/ou de l'orientation du patient permettant de rapprocher de manière optimale une dose administrée au patient de la dose initialement prévue conformément à la première image ;
générer (S3) un ensemble augmenté de deuxièmes images ({rCT1, ..., rCTn}) en appliquant des transformations géométriques {ΔP1, ..., ΔPn} mutuellement différentes dans un espace tridimensionnel à la deuxième image (rCT), et pour chaque espèce de l'ensemble augmenté de deuxièmes images, calculer la réalité de terrain pour la correction requise à partir de l'indication GT de la deuxième image et de la transformation appliquée à cette espèce ;
à l'aide du module de commande basé sur la dose (41), calculer (S4) un vecteur de sortie {ΔPo1, ..., ΔPon} respectif indiquant une correction (xc, yc, zc) de la position et/ou de l'orientation du patient pour chaque paire de la première image (pCT) et d'une espèce (rCTi) de l'ensemble augmenté de deuxièmes images ({rCT1, ..., rCTn}) ;
pour chaque espèce (rCTi) de l'ensemble augmenté de deuxièmes images ({rCT1, ..., rCTn}), calculer (S5) une perte (Li) à partir de la différence entre la correction spécifiée par le vecteur de sortie respectif et le GT respectif ;
mettre à jour (S6) des paramètres du module de commande basé sur la dose (41) pour réduire la perte (Li).
